# EUROPÄISCHE PATENTSCHRIFT

(11) **EP 2 055 261 B1**
(45) Veröffentlichungstag und Bekanntmachung des Hinweises auf die Patenterteilung: **08.03.2017**
(21) Anmeldenummer: 08168240.3
(22) Anmeldetag: 04.11.2008
(51) Int. Cl.: A61C 13/20, F27B 17/02

(54) **MUFFEL ZUR HERSTELLUNG VON ZAHNERSATZTEILEN**
MUFFLE TO MANUFACTURE REPLACEMENT TEETH PARTS
MOUFLE DESTINÉE À LA FABRICATION DE PROTHÈSES DENTAIRES

(30) Priorität: 05.11.2007 AT 17732007
(43) Veröffentlichungstag der Anmeldung: 06.05.2009
(73) Patentinhaber: Ivoclar Vivadent AG, 9494 Schaan (LI)
(72) Erfinder: Harald, Gritsch, 6182 Gries im Sellrain (AT); Max, Wörishofer, 6410 Telfs (AT); Christoph, Zobler, 6071 Aldrans (AT)
(74) Vertreter: Baronetzky, Klaus

(56) Entgegenhaltungen:
- EP-A- 0 231 773
- EP-A- 1 093 769
- DE-A1-102004 013 668
- US-B1- 6 302 186

## Beschreibung

Die Erfindung betrifft eine Muffel zur Herstellung von Zahnersatzteilen, wobei die Muffel ein um die Muffelachse im Wesentlichen rotationssymmetrischer Körper ist. Dazu betrifft die Erfindung eine Vorrichtung und ein Verfahren zur Herstellung einer Muffel. Weiters betrifft die Erfindung einen Pressstempel zur Herstellung von Zahnersatzteilen in Muffeln, wobei der Pressstempel ein im Wesentlichen rotationssymmetrischer Körper ist, eine Vorrichtung und ein Verfahren zur Herstellung eines solchen Pressstempels. Weiters betrifft die Erfindung ein Verfahren und eine Vorrichtung zur Herstellung von Zahnersatzteilen mit erfindungsgemäßen Muffeln und Pressstempeln.

Aus dem Stand der Technik sind bereits zahlreiche Muffeln für die Herstellung von Zahnersatzteilen bekannt, welche im Wesentlichen eine zylindrische Form aufweisen.

Beispielsweise sei hier die DE 20 2005 003 014 U1 genannt, welche eine Muffel mit zwei zylindrischen Bereichen zeigt, wobei die Muffel näherungsweise birnenförmig ausgebildet ist und konvexe und konkave Bereiche aufweist.

In der DE 20 2007 004 265 U1 ist eine Muffel dargestellt, welche rein zylindrisch ausgeführt ist. In ähnlicher Art und Weise wird auch in der DE 10 2004 013 668 A1 eine Muffel dargestellt.

Generell sind bei der Verwendung von Muffeln aus dem Stand der Technik bzw. sonstigen gebräuchlichen Muffeln zahlreiche Probleme bzw. Nachteile bekannt. Das größte Problem besteht darin, große Zahnbrücken in einem Stück herzustellen. Meist werden einzelne Bereiche von Zahnbrücken separat in unterschiedlichen Muffeln hergestellt und anschließend zusammengefügt. Die bisherigen Muffeln bieten zu wenig Platz für größere Zahnersatzteilkonstruktionen. Speziell der Aufnahmeraum für die Ladung in der Muffel ist bei den derzeit bekannten größten Muffeln nur bis zu 10 cm3 groß, was eine maximale Ladung von in etwa 20 g Keramikgewicht erlaubt. Um allerdings weitspannige Brücken überpressen zu können, ist oft mehr Ladung nötig.

Ein Problem, das bei großen Zahnersatzteilen auftaucht, ist die Entstehung von thermischen Spannungsrissen, die von den hohen Spannungen durch das Erhitzen/Abkühlen herrühren und von der Muffel an die Keramik des Zahnersatzteils weitergegeben werden.

Weiters ist durch die kompakte Ausführung der am Markt erhältlichen Keramikpressöfen in diesen für das Herstellen von größeren Zahnersatzteilen in dafür nötigen großen Muffeln nicht ausreichend Platz.

Ein weiteres Problem stellt die Ausführung von Muffeln und Presstempeln aus unterschiedlichem Material dar, da dadurch unterschiedliche Ausdehnungen der einzelnen Materialien vorherrschen und somit vermehrt zu Spannungsrissen auch in der Keramik auftreten.

Aus der EP 1 093 769 A1 ist ein Verfahren und eine Vorrichtung zum Pressen dentaler Elemente bekannt. Ein Rohling wird verflüssigt und ein Pressstempel ist so ausgewählt, dass er einen speziellen wärmeausführenden Koeffizienten hat.

Zusammenfassend ist zum Stand der Technik zu sagen, dass derzeit die Verwendung von Presskeramiken für Anwendungen in der Zahnmedizin auf die Herstellung kleiner Teile beschränkt ist. Bekannte Verfahren zur Herstellung von Zahnersatzteilen sind das Overpressverfahren (mit Keramik überpresstes Zirkon) und das Press-on-Metal-Verfahren, wobei für beide Verfahren gilt, dass nur kleine Zahnersatzteile (bis ca.10 cm3 bzw. bis zu ca. 20 g) hergestellt werden können. Für die gängigen Keramikpressöfen werden derzeit individuell Muffeln vom Hersteller für deren jeweilige Keramikpressöfen gefertigt. Dadurch ist die universelle Verwendbarkeit von handelsüblichen Muffeln praktisch nicht gegeben.

Die Aufgabe der Erfindung liegt nun darin, die vorgenannten Probleme durch das Bereitstellen einer Muffel mit einem großen Aufnahmeraum (größer als 10 cm3) und einem dazupassenden Pressstempel zu lösen.

Dies wird bei der erfindungsgemäßen Muffel dadurch erreicht, dass der - auf die gesamte Oberfläche der äußeren Kontur der Muffel bezogene - Anteil von um die Muffelachse zylindrischen Oberflächenbereichen unter 30 %, vorzugsweise unter 20 % liegt. In verschiedenen Ausführungsvarianten der Muffel kann auch dieser Anteil von um die Muffelachse zylindrischen Oberflächenbereichen bezogen auf die äußere Kontur der Muffel unter 25%, vorzugsweise unter 15% oder unter 20%, vorzugsweise unter 10% liegen. Dabei ist vorgesehen, dass die äußere Kontur der Muffel im Wesentlichen konvex ist.

Die im Wesentlichen konvexe Ausführung bedeutet in diesem Zusammenhang, dass zumindest ein kleiner Bereich, vorzugsweise im Sockelbereich der Muffel, auch konkav ausgeführt sein kann, wobei dieser Bereich der Zentrierung der Muffel im Keramikpressofen dient. Je nach Art des Ofens kann der konkave Bereich auch angepasst oder weggelassen werden. Generell bietet die konvexe Ausführung der Muffel den Vorteil, dass Spannungsrisse weitestgehend ausgeglichen bzw. vermieden werden.

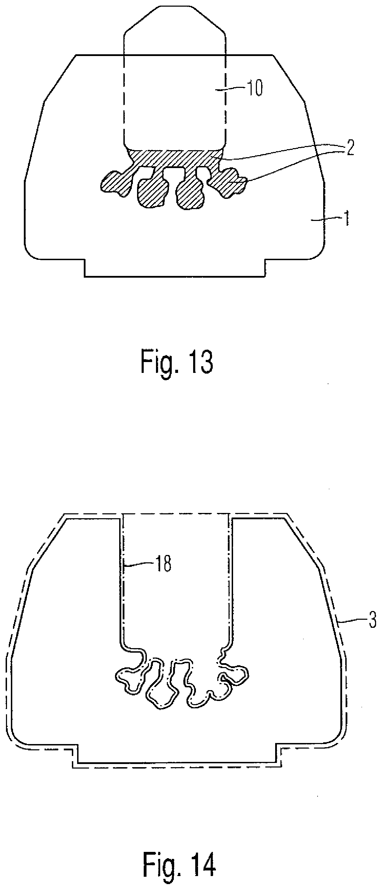

Die äußere Kontur der Muffel ist der Bereich, der nicht durch den Aufnahmeraum für die Ladung bzw. den Pressstempel und die Negativform des Zahnersatzteiles gebildet wird. In diesem Zusammenhang soll aus Figur 14, welche weiter unten beschrieben wird, verwiesen werden.

In einer besonderen Ausführungsform ist vorgesehen, dass die äußere Kontur der Muffel mindestens zwei kegelstumpfförmige Bereiche aufweist, wobei die kegelstumpfförmigen Bereiche unterschiedliche Öffnungswinkel (α, β) aufweisen. Diese Ausführungsform führt dazu, dass die Muffel den Raum im Keramikpressofen besonders im oberen Bereich optimal ausnutzen kann, wodurch die Durchwärmeigenschaft der Muffel verbessert wird.

Vorzugsweise soll auch vorgesehen sein, dass die Muffel zumindest einen zylindrischen Bereich aufweist. Besonders im breitesten Bereich der Muffel dient die zylindrische Ausführung dazu, für große Zahnersatzteile genügend Platz zu bieten.

Weiters soll bevorzugt vorgesehen sein, dass die Muffel zumindest einen gekrümmten Oberflächenbereich aufweist. Dieser zumindest eine gekrümmte Bereich dient vor allem dem Ausgleich von Spannungsrissen. Möglichst alle scharfen Kanten, die beim Stand der Technik für Muffeln entstehen, sollten erfindungsgemäß durch eine gekrümmte Oberflächenform vermieden werden.

Trotz der vorteilhaften konvexen Ausführung der Muffel soll vorgesehen sein, dass die äußere Kontur der Muffel im oberen und unteren Bereich abgeflacht ist. Obwohl durch eine vollkonvexe Ausführung der Muffel (zB eiförmig oder kugelförmig) Spannungsrissen am besten vorgebeugt werden kann, ist die abgeflachte Ausführung der Muffel beim Pressvorgang wichtig, da dadurch die Presskräfte besser und gleichmäßiger von oben nach unten weitergegeben werden können. Vor allem der abgeflachte untere Bereich dient dazu, die Presskräfte großflächig weiterzugeben, da bei einer abgerundeten Ausführung der unteren Seite (Sockel) die Stabilität der gesamten Muffel während des Pressvorganges beeinträchtigt ist.

Besonders vorteilhaft soll für die abgeflachten Bereiche vorgesehen sein, dass zwischen 5 und 55 %, vorzugsweise zwischen 30 und 45 %, der äußeren Kontur in rechtem Winkel zur Muffelachse ausgebildet sind.

Eine besonders vorteilhafte Ausführungsform sieht vor, dass der rotationssymmetrische Körper der Muffel - bezogen auf die äußere Kontur und von oben nach unten geseheneinen kegelstumpfförmigen Körper mit einem Öffnungswinkel (α), einen kegelstumpfförmigen Körper mit einem Öffnungswinkel (β), wobei α > β gilt, einen zylindrischen Körper und einen rotationssymmetrischen Körper mit gekrümmter Oberfläche aufweist. In zahlreichen Versuchen und durch verschiedene Möglichkeiten der Ausbildung der Muffel, ist diese Form der Muffel als besonders resistent gegenüber Spannungsrissen ausfindig gemacht worden. Im Speziellen beträgt der Öffnungswinkel β 79° und der Öffnungswinkel α 62°. Zusätzlich zu dieser vorteilhaften Ausführung kann vorgesehen sein, dass die Muffel einen weiteren zylindrischen Körper, der vorzugsweise im unteren Bereich der Muffel angeordnet ist, aufweist. Dieser Bereich dient dazu, wie bereits erwähnt, die Position der Muffel im KeramikPressofen beim Pressen zu fixieren.

Eine vorteilhafte Ausführungsform, welche sich nicht auf die vorgenannten möglichen Ausführungsformen bezieht, sieht vor, dass der Anteil von um die Muffelachse zylindrischen Oberflächenbereichen bezogen auf die äußere Kontur der Muffel bei 0 % liegt. Diese Ausführungsform bietet einen besonderen Vorteil im Hinblick auf die Spannungsrissresistenz.

Zur Herstellung einer erfindungsgemäßen Muffel wird Schutz für eine Vorrichtung begehrt, in welcher eine flüssige Einbettmasse in einem Hohlraum zu einer Muffel aushärtbar ist. Der Hohlraum dieser Vorrichtung bildet die Negativform der erfindungsgemäßen Muffel. Im Speziellen wird der Hohlraum der Vorrichtung durch zwei Formgeber, einem auf einem Anstiftdorn eines Formgebers befindlichen rückstandslos ausbrennbaren Medium und einem Silikonring gebildet. Auch andere Formgebungsteile zur Bildung eines Hohlraumes für die Herstellung einer Muffel sollen im Sinne der Erfindung nicht aufgeschlossen werden.

In diesem Zusammenhang soll auch ein Verfahren geschützt werden, bei dem in einer Vorrichtung nach den vorgenannten Ausführungsformen eine Muffel aushärtbar ist.

Einen zweiten unabhängigen Aspekt bildet der Pressstempel, welcher die Ladung in die Negativform des Zahnersatzteils in einer Muffel presst. Bekannt sind dabei bereits Pressstempel zur Herstellung von Zahnersatzteilen in Muffeln, wobei der Pressstempel ein im Wesentlichen rotationssymmetrischer Körper ist.

Die Aufgabe eines Pressstempels besteht darin, eine - vorzugsweise große und über 20 g schwere - Ladung in die Negativform des Zahnersatzteiles in der Muffel zu pressen.

Bei diesem Pressstempel wird dies dadurch gelöst, dass das Höhen-Grundflächendurchmesser-Verhältnis des Pressstempels im Bereich zwischen 2:1 bis 1:4, vorzugsweise bei etwa 1:1, liegt.

Vorteilhaft kann darüber hinaus vorgesehen sein, dass der Durchmesser des Pressstempels im oberen Bereich schmäler als im unteren Bereich ist. Diese Ausführung ist der Kraftübertragung vom Geber über den Pressstempel auf die Einbettmasse in einem relativ breiten Aufnahmeraum (über 10 mm Durchmesser, vorzugsweise ca. 27 mm Durchmesser) förderlich.

Derzeit sind auf dem Markt Stempel erhältlich, die einen Durchmesser von ca. 10 mm und eine Höhe von ca. 30 bis 40 mm aufweisen, also nur auf einem am Markt erhältlichen Rohling direkt pressen, wobei mehrere Rohlinge übereinander gestellt sein können. Durch die vorteilhafte Ausführung des Pressstempels im unteren Bereich von über 10 mm, vorzugsweise von 27 mm, kann nicht nur auf einen einzelnen Rohling gepresst werden, sondern auch auf mehrere nebeneinander angeordnete Rohlinge. Dadurch ist das Verpressen von neun ca. 3 g schweren Rohlingen im Aufnahmeraum der Muffel durch den Pressstempel möglich, wodurch eine bis zu 14-gliedrige Brückenkonstruktion in einem Arbeitsschritt hergestellt werden kann. Vorzugsweise bildet der Pressstempel die gleiche Form wie der Anstiftdorn und ist zumindest in einem Oberflächenbereich gekrümmt.

Besonders vorteilhaft kann vorgesehen sein, dass der Durchmesser des oberen Bereiches im Wesentlichen dem Durchmesser eines Gebers eines Keramikpressofens und im unteren Bereich im Wesentlichen dem Durchmesser des Aufnahmeraumes entspricht.

Besonders vorteilhaft ist des Weiteren vorgesehen, dass der - auf die gesamte Oberfläche des Pressstempels bezogene - Anteil von um die Pressstampelachse zylindrischen Oberflächenbereichen unter 80 %, vorzugsweise unter 60 % liegt. Ebenfalls ist dabei zu berücksichtigen, dass der Pressstempel im Wesentlichen konvex ist. Des Weiteren kann vorgesehen sein, dass der Pressstempel mindesten einen kegelstumpfförmigen Bereich aufweist.

Im Besonderen wird des Weiteren Schutz für eine Vorrichtung zur Herstellung eines vorgenannten Pressstempels zur Verwendung in einer vorgenannten Muffel begehrt, wobei eine flüssige Einbettmasse in einem Hohlraum der Vorrichtung zu einem Pressstempel aushärtbar ist. Vorteilhaft soll dabei vorgesehen sein, dass der Hohlraum der Vorrichtung die Negativform des Pressstempels bildet.

Eine Ausführungsform sieht dabei vor, dass der Hohlraum der Vorrichtung durch einen Silikonkörper und einem Formgeber gebildet wird. Vorzugsweise kontaktieren sich der Silikonkörper und der Formgeber in dem Bereich, in dem der kegelstumpfförmige Bereich des Pressstempels in den zylinderförmigen Bereich des Pressstempels übergeht.

Schutz wird in diesem Zusammenhang auch für ein Verfahren zur Herstellung eines Pressstempels begehrt, wobei in einer vorgenannten Vorrichtung zur Herstellung von Pressstempeln der Pressstempel aushärtbar ist.

Schutz wird auch für ein Verfahren zur Herstellung von Zahnersatzteilen begehrt, wobei in einem Vorwärmofen eine Muffel nach einem der Ansprüche 1 bis 8 und ein Presstempel erhitzt wird, eine Ladung für das Zahnersatzteil in einen Aufnahmeraum der Muffel gegeben wird, der Aufnahmeraum samt Ladung mit dem Pressstempel bedeckt wird, die Muffel, die Ladung und der Pressstempel in einen Keramikpressofen, welchen ein Vakuum erzeugt, gegeben wird, die Ladung durch einen Pressstempel in eine durch das Medium in der Muffel gebildete Negativform bei ca. 930° C gepresst wird.

Von besonderer Bedeutung ist dabei, dass die Muffel und der Pressstempel aus derselben Einbettmasse bestehen. Die Einbettmasse wird von Zahntechnikern bei der Herstellung von der Muffel angerührt, wobei meist eine gewisse Restmasse übrig bleibt. Diese Restmasse kann erfindungsgemäß in einer Vorrichtung zu einem Pressstempel ausgehärtet werden, welcher somit die exakt selbe Materialzusammensetzung wie die Muffel aufweist. Da der Pressstempel und die Muffel für das Pressen des Zahnersatzteils im Keramikpressofen gleichzeitig erhitzt werden, ist die Verwendung ein und derselben Einbettmasse für die Herstellung dieser beiden Komponenten wiederum für das Unterbinden von Spannungsrissen von immensem Vorteil.

Ein weiterer Aspekt der Erfindung betrifft eine Vorrichtung zur Herstellung von Zahnersatzteilen mit einer Muffel, insbesondere nach einem der Ansprüche 1 bis 8, und einem Pressstempel, wobei in der Muffel eine Negativform der Zahnersatzteile und ein Aufnahmeraum für eine zu verpressende Ladung, insbesondere für eine Einbettmasse, ausgebildet sind. Hierbei ist vorgesehen, dass der Aufnahmeraum ein Volumen von über 11 cm³, vorzugsweise von über 12,5 cm³, aufweist.

Vorteilhaft kann bei diesem Verfahren vorgesehen sein, dass der Aufnahmeraum ein Volumen zwischen 15 und 30 cm³, vorzugsweise zwischen 17 und 23 cm³ aufweist. Beim bekannten Pressmaster weist der Aufnahmeraum ein Volumen von ca. 10 cm³ auf. Im Gegensatz dazu bietet die erfindungsgemäße Muffel, vor allem beim Einsatz von der Press-On-Metal-Technik für die Herstellung von weitspannigen Brücken einen größeren Aufnahmeraum und somit eine höhere Ladungskapazität. Die Ladungskapazität von neun Rohlingen entspricht einem Volumen von 10,86 cm3. Das Gewicht von 9 Rohlingen beträgt bei PoM-Rohlingen 26,6 g, bei E-mex-press-Rohlingen 28,5 g und bei E-mex-zirpressRohlingen 25,6 g. Die Dichteunterschiede entstehen aufgrund unterschiedlicher Zusammensetzung der Rohlinge, so besteht zum Beispiel der E-mex-Press-Rohling aus Lithiumdysilikat.

In einer besonderen Ausführung weist ist eine erfindungsgemäße Muffel zur Herstellung von Zahnersatzteilen ein um die Muffelachse im Wesentlichen rotationssymmetrischer Körper, wobei der - auf die gesamte Oberfläche der äußeren Kontur (3) der Muffel bezogene - Anteil von um die Muffelachse zylindrischen Oberflächenbereichen unter 30 %, vorzugsweise unter 25 %, insbesondere unter 20 %, bevorzugt unter 15 % und besonders bevorzugt unter 10 %, liegt.

In einer Weiterbildung der Erfindung liegt der Anteil von um die Muffelachse zylindrischen Oberflächenbereichen bezogen auf die äußere Kontur der Muffel unter 25 %, vorzugsweise unter 15 %.

In einer Weiterbildung der Erfindung liegt der Anteil von um die Muffelachse zylindrischen Oberflächenbereichen bezogen auf die äußere Kontur der Muffel unter 20 %, vorzugsweise unter 10 %.

In einer Weiterbildung der Erfindung ist die äußere Kontur der Muffel im Wesentlichen konvex.

In einer Weiterbildung der Erfindung weist die äußere Kontur der Muffel mindestens zwei kegelstumpfförmige Bereiche auf.

In einer Weiterbildung der Erfindung weisen die kegelstumpfförmigen Bereiche unterschiedliche Öffnungswinkel auf.

In einer Weiterbildung der Erfindung weist die Muffel zumindest einen zylindrischen Bereich auf.
In einer Weiterbildung der Erfindung weist die Muffel zumindest einen gekrümmten Oberflächenbereich auf.

In einer Weiterbildung der Erfindung ist die äußere Kontur der Muffel im oberen und unteren Bereich abgeflacht.

In einer Weiterbildung der Erfindung sind zwischen 5 und 55 %, vorzugsweise zwischen 30 und 45 %, der äußeren Kontur in rechtem Winkel zur Muffelachse ausgebildet.

In einer Weiterbildung der Erfindung weist der rotationssymmetrische Körper der Muffel - bezogen auf die äußere Kontur und von oben nach unten gesehen - einen kegelstumpfförmigen Körper mit einem ersten Öffnungswinkel, einen kegelstumpfförmigen Körper mit einem zweiten Öffnungswinkel, wobei der erste Öffnungswinkel größer als der zweite Öffnungswinkel ist, einen zylindrischen Körper und einen rotationssymmetrischen Körper mit gekrümmter Oberfläche auf.

In einer Weiterbildung der Erfindung weist die Muffel einen weiteren zylindrischen Körper auf, der vorzugsweise im unteren Bereich der Muffel angeordnet und als Sockel ausgeführt.

In einer Weiterbildung der Erfindung liegt der Anteil von um die Muffelachse zylindrischen Oberflächenbereichen bezogen auf die äußere Kontur der Muffel bei 0 %.

In einer Weiterbildung der Erfindung ist eine flüssige Einbettmasse in einem Hohlraum zu einer Muffel aushärtbar.

In einer Weiterbildung der Erfindung bildet der Hohlraum der Vorrichtung die Negativform der Muffel.

In einer erfindungsgemäßen Vorrichtung zur Herstellung einer erfindungsgemäßen Muffel ist eine flüssige Einbettmasse in einem Hohlraum zu einer Muffel aushärtbar.

In einer Weiterbildung der Erfindung bildet der Hohlraum der Vorrichtung insbesondere die Negativform der Muffel.

In einer Weiterbildung der Erfindung wird der Hohlraum (5a) der Vorrichtung durch zwei Förmgeber, einem auf einem Anstiftdorn eines Formgebers befindlichen rückstandslos ausbrennbaren Medium und einen Silikonring gebildet.

In einem erfindungsgemäßen Verfahren zur Herstellung einer erfindungsgemäßen Muffel ist die Muffel in einer erfindungsgemäßen Vorrichtung zur Herstellung der erfindungsgemäßen Muffel aushärtbar.

Ein Pressstempel zur Herstellung von Zahnersatzteilen in Muffeln ist ein im Wesentlichen rotationssymmetrischer Körper und weist ein Höhen-Grundflächendurchmesser-Verhältnis (H:G) im Bereich zwischen 2:1 bis 1:4, vorzugsweise bei etwa 1:1 auf.

Der Durchmesser des Pressstempels ist im oberen Bereich schmäler als im unteren Bereich.

Der Durchmesser entspricht im oberen Bereiches im Wesentlichen dem Durchmesser eines Gebers eines Keramikpressofens und im unteren Bereich im Wesentlichen dem Durchmesser des Aufnahmeraumes.

Der - auf die gesamte Oberfläche des Pressstempels bezogene - Anteil von um die Pressstampelachse zylindrischen Oberflächenbereichen unter 80 %, vorzugsweise unter 60 %.

Der Pressstempel ist im Wesentlichen konvex.

In einer Weiterbildung der Erfindung weist der Pressstempel mindestens einen kegelstumpfförmigen Oberflächenbereich auf.
In einer Weiterbildung der Erfindung weist der Pressstempel einen gekrümmten Oberflächenbereich auf.

In einer Weiterbildung weist der rotationssymmetrische Körper des Pressstempels - von oben nach unten gesehen - einen kegelstumpfförmigen Körper, einen zylindrischen Körper und einen rotationssymmetrischen Körper mit gekrümmter Oberfläche auf.

Eine erfindngsgemäße Vorrichtung zur Herstellung eines Pressstempels für das Pressen von Zahnersatzteilen in einer erfindungsgemäßen Muffel weist einen Hohlraum auf, in dem eine flüssige Einbettmasse zu einem Pressstempel aushärtbar ist.

In einer Weiterbildung bildet der Hohlraum der Vorrichtung die Negativform des Pressstempels.

In einer Weiterbildung der Erfindung wird der Hohlraum der Vorrichtung durch einen Silikonkörper und einen Formgeber gebildet.

In einem Verfahren zur Herstellung eines Pressstempels ist der Presstempel in einer erfindungsgemäßen Vorrichtung zur Herstellung des Pressstempels aushärtbar.

In einem erfindungsgemäßen Verfahren zur Herstellung von Zahnersatzteilen wird in einem Vorwärmofen eine erfindungsgemäße Muffel und ein Presstempel erhitzt, eine Ladung für das Zahnersatzteil in einen Aufnahmeraum der Muffel gegeben, der Aufnahmeraum samt Ladung mit dem Pressstempel bedeckt, die Muffel, die Ladung und der Pressstempel in einen Keramikpressofen, welcher ein Vakuum erzeugt, gegeben und die Ladung durch den Pressstempel in eine durch das Medium in der Muffel gebildete Negativform gepresst.

In einer Weiterbildung der Erfindung bestehen die Muffel und der Pressstempel aus derselben Einbettmasse.

In einer erfindungsgemäßen Vorrichtung zur Herstellung von Zahnersatzteilen mit einer erfindungsgemäßen Muffel und einem Pressstempel sind in der Muffel eine Negativform der Zahnersatzteile und ein Aufnahmeraum für eine zu verpressende Ladung, insbesondere für eine Zahnersatzteilmasse, ausgebildet, wobei der Aufnahmeraum ein Volumen von über 11 cm³, vorzugsweise von über 12,5 cm³, aufweist.

In einer Weiterbildung der Erfindung weist Aufnahmeraum ein Volumen zwischen 15 und 30 cm³, vorzugsweise zwischen 17 und 23 cm³ aufweist.

Weitere Einzelheiten und Vorteile der vorliegenden Erfindung werden anhand der folgenden Figurenbeschreibung unter Bezugnahme auf die in den Zeichnungen dargesellten Ausführungsbeispiele im Folgenden näher erläutert. Darin zeigen:
- Fig.1: einen Querschnitt der erfindungsgemäßen Muffel mit einem beispielhaften Pressstempel und Ladung vor dem Pressen,
- Fig.2: einen Querschnitt der Muffel mit Pressstempel beim Pressen,
- Fig.3,4,5 und 6: die Herstellungsschritte der Muffel,
- Fig.7 und 8: die Herstellung des Pressstempels,
- Fig.9: einen Querschnitt der Vorrichtung zur Herstellung eines Pressstempels,

- Fig.10: den Pressstempel
- Fig.11: den Pressstempel und die Muffel mitsamt Ladung vor dem Pressen,
- Fig.12: den Keramikpressofen, die Muffel und den Pressstempel vor dem Pressen,
- Fig.13: das eingepresste Zahnersatzteil,
- Fig.14: einen Querschnitt mit Kennzeichnung der äußeren und inneren Kontur,
- Fig.15: eine vertikale Draufsicht auf den Aufnahmeraum samt Ladung,
- Fig.16: eine Ansicht des Aufnahmeraumes samt Ladung und
- Fig.17a bis 17e: Querschnitte von Pressstempeln.

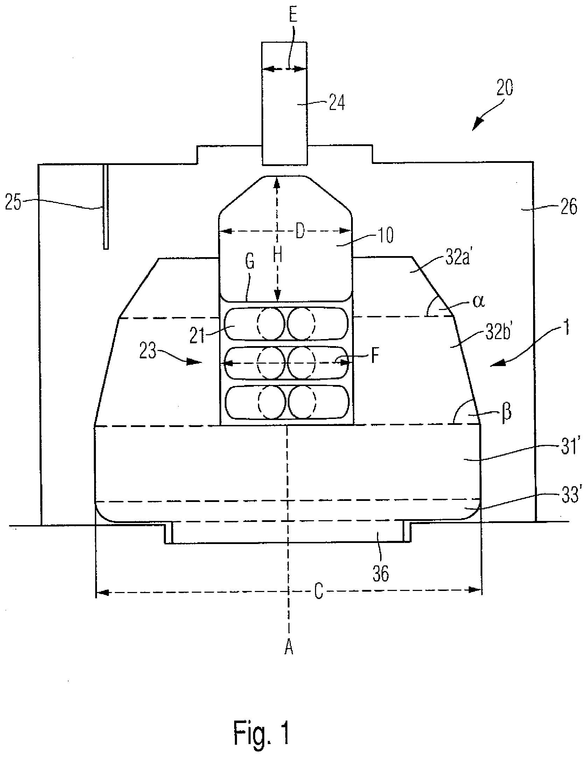

In Fig. 1 ist ein Querschnitt der konkreten Ausführung einer erfindungsgemäßen Muffel dargestellt. In diesem Fall hat der Ofenraum 26 des Keramikpressofens 20 einen Durchmesser von 9, 8 cm (als Keramikpressofen dient beispielsweise der Ofen EP5000 der Firma Ivoclar). Die Muffel weist von oben nach unten gesehen im obersten Bereich einen kegelstumpfförmigen Körper 32a' mit einem Öffnungswinkel α von 62a auf. Anschließend folgt ein kegelstumpfförmiger Körper 32b' mit einem Öffnungswinkel β von 79°. Diese beiden zusammen weisen in diesem Fall eine Höhe von 4 cm auf. Anschließend folgt ein zylindrischer Körper 31' und darauffolgend ein gekrümmter rotationssymmetrischer Körper 33', welche zusammen eine Höhe von 2,7 cm aufweisen. Für das Einstellen der Muffel in diese Art eines Presskeramikofens ist am untersten Ende noch ein Sockel 36 vorgesehen, welcher die gesamte Muffel während des Pressvorgangs in Position hält. Der Durchmesser der gesamten Muffel 1 beträgt im zylindrischen Bereich 31 8 cm. Der Aufnahmeraum 23 bildet in seinem Umfang die innere Kontur 18 der Muffel 1 (siehe Fig. 14), wobei der Aufnahmeraum 23 in diesem Fall dieselbe Höhe einnimmt, wie die beiden oberen kegelstumpfförmigen Bereiche 32a und 32b und somit 4 cm hoch ist (wobei dies nicht zwingend vorgesehen sein muss). In diesem Aufnahmeraum 23 ist die Ladung 21, bestehend aus neun Rohlingen, dargestellt, wobei eine maximale Füllung bedeutet. Der Durchmesser D des Pressstempels 10 ist gleich groß wie der Durchmesser F des Aufnahmeraums 23, wodurch der Pressstempel genau in den Aufnahmeraum 23 passt. Der Pressstempel weist eine Höhe von 3 cm auf, wobei im obersten Bereich des Pressstempels 10 der Geber 24 des Keramikpressofens 20 mit einem Durchmesser E von 1 cm auf dem Pressstempel 10 aufschlägt. Im linken Bereich ist ein Thermostat 25 zur Regelung der Innentemperatur dargestellt. Die Negativform der Zahnersatzteile 2 ist nicht dargestellt.

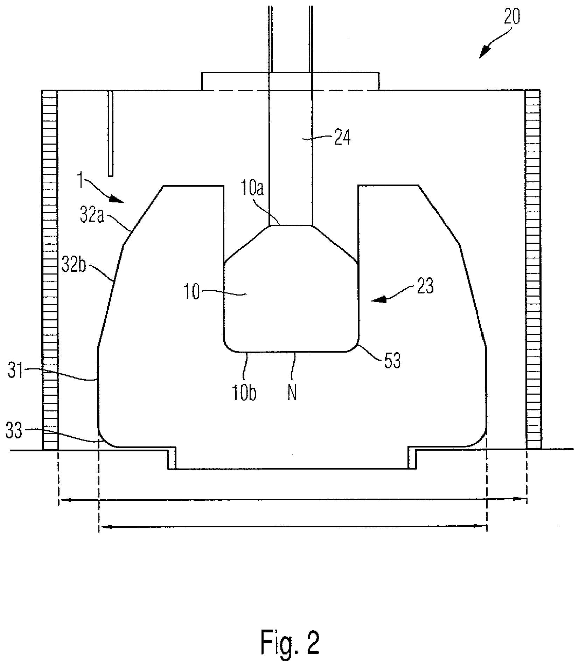

Fig.2 zeigt dieselbe Muffel 1 wie Fig.1 diesmal nach Ausführung des Pressvorgangs. Hierbei ist der Geber 24 des Presskeramikofens 20 ausgefahren, wodurch der Pressstempel 10 am Nullpunkt N des Aufnahmeraumes 23 anschlägt. Somit ist die gesamte Ladung 21 in den Hohlraum der Zahnersatzteile (nicht dargestellt) eingepresst. Der untere abgerundete Bereich 53 des Pressstempels 10 ist passgenau mit der abgerundeten Fläche des Aufnahmeraums 23 ausgeführt. In diesem Fall sind die kegelstumpfförmigen Oberflächenbereiche 32a und 32b, der zylindrische Oberflächenbereich 31 und der gekrümmte Oberflächenbereich 33 gekennzeichnet. Die Schräge bzw. Krümmung im unteren Bereich 10b des Pressstempels 10 wirkt möglichen Verklemmungen des Pressstempels 10 im Aufnahmeraum 23 entgegen und dient der Spannungshemmung sowie dem leichteren "Einfädeln" des Pressstempels 10 in den Aufnahmeraum 23.

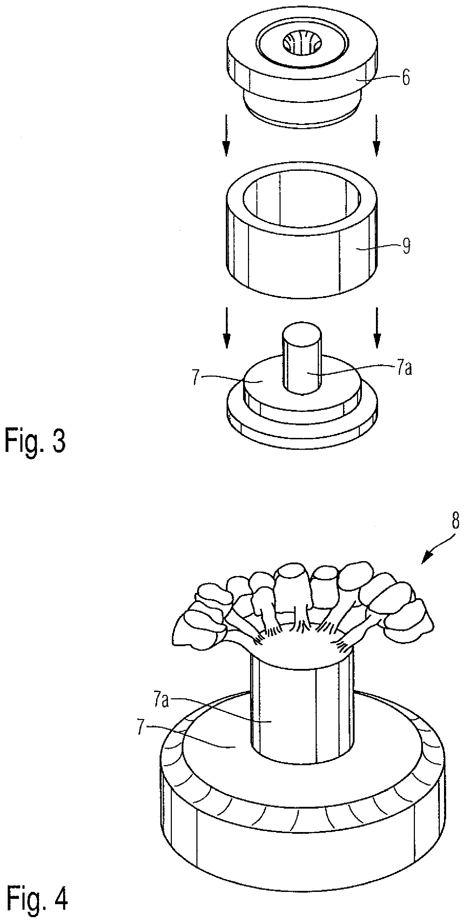

Fig. 3 zeigt den Formgeber 6 mit der Trichteröffnung, den Silikonring 9 und den Formgeber 7 mit Anstiftdorn 7a, welche einen Teil des Hohlraums 5a zur Aufnahme der flüssigen Einbettmasse 4 für die Herstellung der Muffel 1 bilden.

In Fig. 4 ist dazu noch eine genauere Darstellung des Formgebers 7 mit dem Anstiftdorn zu sehen, wobei auf dem Anstiftdorn 7a das rückstandslos aushrennbare Medium 8, welches die Positivform des Zahnersatzteils 2 bildet, angeordnet ist.

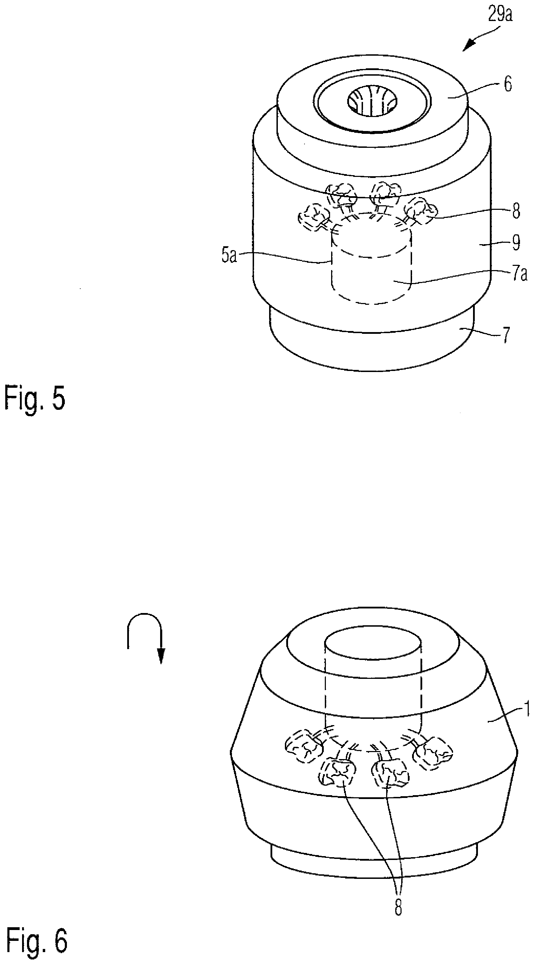

In Fig. 5 sind nun diese vier Bestandteile zur Bildung des Hohlraums 5a für die Herstellung der Muffel 1 in zusammengesetzter Form ersichtlich. Dieser Hohlraum 5a wird mit der flüssigen Einbettmasse 4 gefüllt. In ca. 10 bis 25 Minuten härtet die Einbettmasse 4 aus, wonach der Formgeber 6, der Silikongeber 9 und der Formgeber 7 entfernt werden können, wobei der Formgeber 7 mit dem Anstiftdorn 7a aufgrund der rotationssymmetrischen Ausführung nicht nur herausgezogen sondern vorzugsweise herausgedreht werden kann, ohne das Auftreten von Spannungsrissen befürchten zu müssen.

Nach Entfernen dieser drei eben genannten Komponenten bleibt, wie in Fig. 6 dargestellt, eine "gestürzte" Muffel 1 übrig, wobei im Bereich für die Zahnersatzteile 2 noch das rückstandslos ausbrennbare Medium 8 den Hohlraum in der Muffel 1 ausfüllt. Beim Aufheizen und Aushärten der Muffel 1 im Vorwärmofen 19 (hier nicht dargestellt) brennt das Medium 8 (welches vorzugsweise organisches bzw. hochreines Wachs ist) rückstandslos aus.

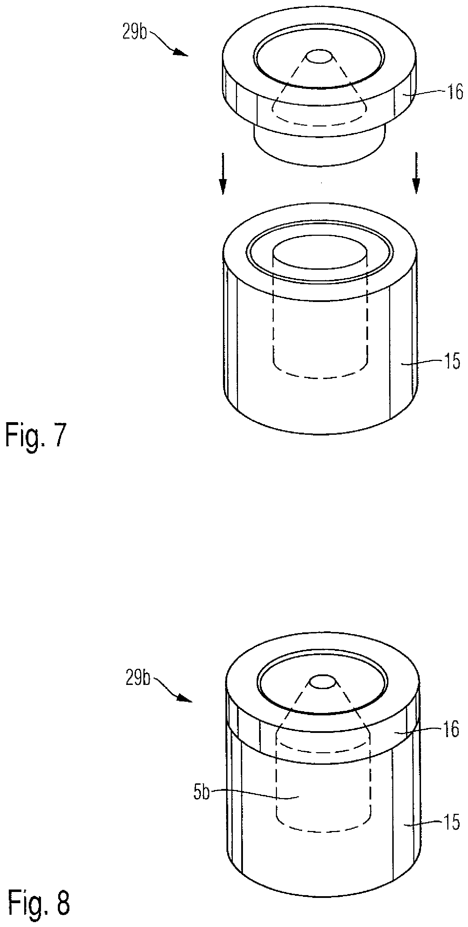

Fig. 7 zeigt nun die Vorrichtung 29b zur Herstellung des Pressstempels 10, wobei diese Vorrichtung aus dem Formgeber 16 und dem Silikonkörper 15 besteht. In Fig. 8 ist dargestellt, wie diese beiden Komponenten den Hohlraum 5b für den Pressstempel 10 bilden.

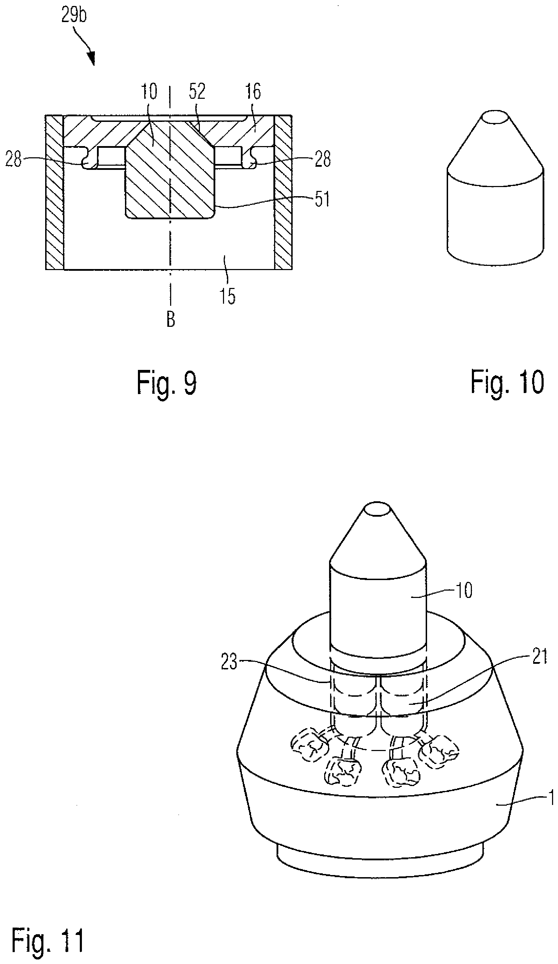

In Fig. 9 ist ein Querschnitt dieser Vorrichtung 29b durch die Pressstempelachse B gezeigt. Hierbei ist die spezielle Ausführung und das ineinander greifen von Formgeber 16 und in den Silikonkörper 15 mittels der Formgeberlaschen 28 dargestellt. Der Kontaktbereich von Silikonkörper 15 und Formgeber 16 bildet genau den Übergang vom kegelstumpfförmigen Bereich 52 des Pressstempels 10 in den zylinderförmigen Bereich 51 des Pressstempels 10, wodurch eine absolut ebene Oberfläche des Pressstempels 10 garantiert ist. Der Durchmesser der gesamten Vorrichtung 29b beträgt 6,8 cm und die Höhe 5,5 cm. Der Pressstempel 10 hat einen Durchmesser D von 2,7 cm.

Fig.10 zeigt nun eine perspektivische Ansicht der im Hohlraum 5b der Vorrichtung 29b zur Herstellung des Pressstempels 10 ausgehärteten flüssigen Einbettmasse 4.

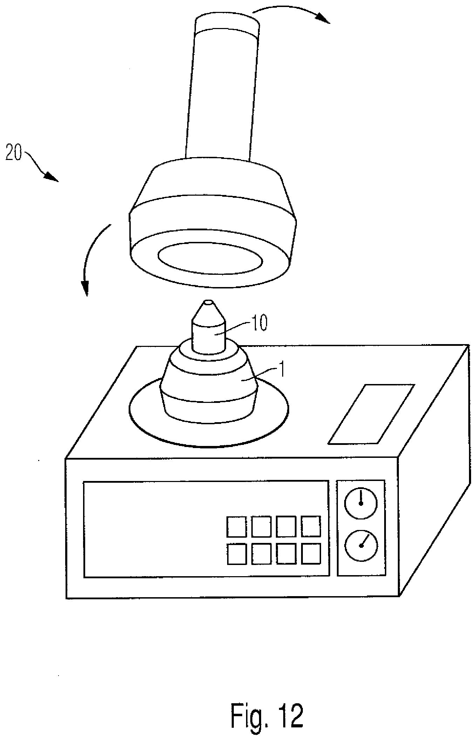

Fig. 11: Beim Herstellungsverfahren für ein Zahnersatzteil 2 wird der Pressstempel 10 und die Muffel 1, die aus ein und derselben flüssigen Einbettmasse 4 hervorgehen, zusammen im Vorwärmofen 19 (nicht dargestellt) auf ca. 850° C erhitzt. Nach ca. 90-minütigem Erhitzen dieser beiden Komponenten werden diese aus dem Vorwärmofen 19 herausgegeben mit der gewünschten Ladung 21 im Aufnahmeraum 23 der Muffel 1 bestückt und unmittelbar danach in den Keramikpressofen 20 (siehe Fig. 12) gegeben, in welchem der Pressvorgang durchgeführt wird. Dieser Pressvorgang läuft weitestgehend automatisch ab und dauert zwischen 10 und 40 Minuten, wobei die Ladung 21 in auf ca. 910° bis 950° C erwärmten Keramikpressofen 20 zähflüssig wird und über den Geber 24 und den Pressstempel 10 in die Negativform der Zahnersatzteile 2 gepresst wird, wobei im Keramikpressofen 20 ein Vakuum herrscht.

Fig.12 zeigt den Keramikpressofen 20 in geöffnetem Zustand vor dem Pressen mit der darin befindlichen Muffel 1 und dem Presstempel 10.

Fig. 13 zeigt einen Querschnitt der Muffel 1 und des Pressstempels 10 mit dem aus gehärtetem Keramik bestehenden Zahnersatzteil 2.

Zum besseren Verständnis ist in Fig. 14 die äußere Kontur 3 der Muffel 1 strichliert dargestellt. Die äußere Kontur 3 bildet sozusagen den äußeren Umfang der Muffel 1, während die innere Kontur 18 den inwärts gewandten Oberflächenbereich der Muffel bildet. Die innere Kontur 18 wird durch den Anstiftdorn 7a des Formgebers 7 und das Medium 8 gebildet.

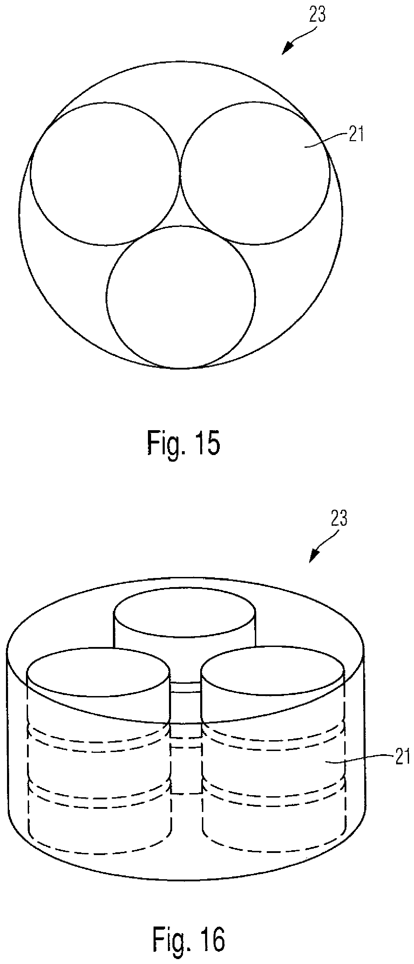

Fig. 15 zeigt eine Draufsicht auf den Aufnahmeraum 23 samt Ladung 21 von oben. Hierbei sind die derzeit gängigen Rohlinge mit einem Durchmesser von ca. 1 cm dargestellt. Auch andere Arten und Größen von Einbettmassen bzw. Rohlingen sind für den Gebrauch in einer erfindungsgemäßen Muffel 1 möglich, so würde z. B. eine Rohling, der an die Form des erfindungsgemäßen Aufnahmeraums 23 angepasst und diese exakt ausfüllt, das Füllvolumen um ca. 67 % besser ausnutzen. D. h., dass der Aufnahmeraum 23 bei einem Durchmesser von 2,7 cm und einer Höhe von 3 cm, ein Volumen von 17,18 cm³ aufweist, während 9 derzeit gängige Rohlinge (siehe Fig. 16) mit einem Durchmesser von ca. 1,2 cm und einer Höhe von 1 cm ein Volumen von ca. 10, 18 cm³ haben. Auch bei eventueller in Zukunft möglicher Verwendung von einer Art Granulat anstatt Rohlingen, bietet der erfindungsgemäße Aufnahmeraum 23 ausreichend Volumen um bis zu 14-gliedrige Brückenkonstruktionen in einem Stück herzustellen. Vor allem für Konstruktionen, die kein Metallgerüst aufweisen, ist wesentlich mehr Keramikmasse erforderlich, was durch die erfindungsgemäße Ausführung von Muffel und Pressstempel unterstützt wird. Bei der Herstellung der Zahnersatzteile 2 in bestimmten Keramikpressöfen 20 kann der Aufnahmeraum 23 auch eine Höhe von bis zu 4 cm aufweisen und somit ein Füllvolumen von bis zu 22,9 cm³ bieten. Hierdurch könnten auch mehr als 9 derzeit übliche Rohlinge verpresst werden.

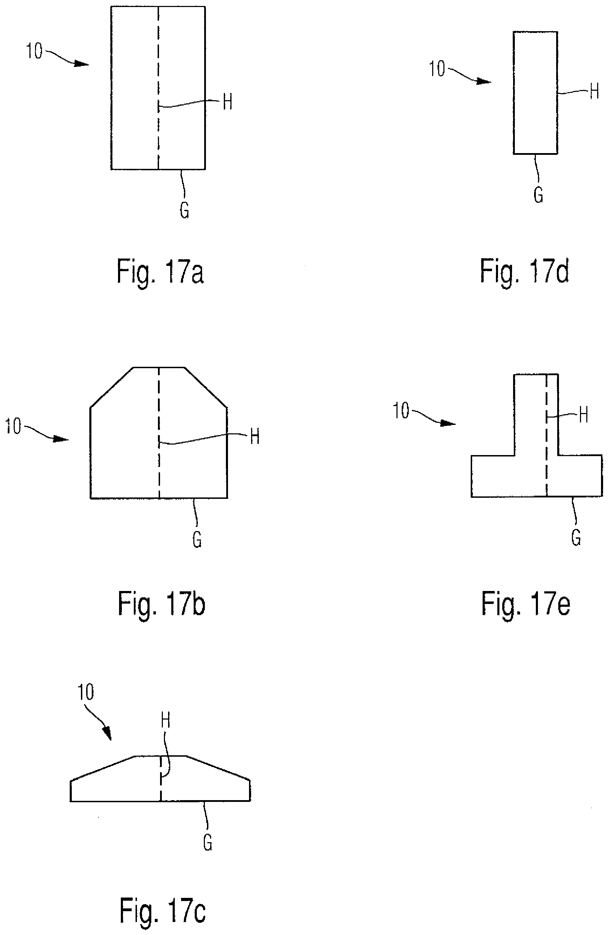

Fig.17a bis 17c zeigt Querschnitte möglicher Pressstempel 10, wobei Fig. 17a ein Höhen-Grundflächendurchmesser-Verhättnis (H:G) von 2:1 aufweist, Fig. 17b von 1:1 und
Fig. 17c von 1:4. Gemein ist diesen unterschiedlichen Ausführungen, dass der Pressstempel 10 eine größere Pressfläche (Grundfläche) aufweist als Fig. 17d, welche den Stand der Technik mit einem Höhen-Grundflächendurchmesser-Verhältnis von 3:1 und einem Durchmesser D von ca. 10 mm zeigt. Fig. 17e zeigt eine ebenfalls mögliche Ausführung eines Pressstempels 10 mit dem Verhältnis H:G von ca. 1:1. Dazu ist zu erwähnen, dass bereits ein Pressstempel 10 mit einem solchen Querschnitt existiert, dieser jedoch nicht rotationssymmetrisch ausgeführt ist, wodurch vor allem beim Entfernen des Pressstempels 10 nach dem Verpressen aus der Muffel 1 Probleme (eventuell Spannungsrisse, Abbrechen, Verklemmen usw.) auftreten. Da der Pressstempel 10 im untersten Bereich 53 abgerundet bzw. gekrümmt ist, gilt als Grundflächendurchmesser G der Bereich oberhalb des abgerundeten Bereiches 53. In anderen Worten kann der breiteste Bereich des Pressstempels 10 als Grundflächendurchmesser G herangezogen werden. Auch eine sehr flache Ausführung des Pressstempels 10 (H:G ca. 1:5 bis 1:100) ist für das Durchführen des Pressvorganges möglich, jedoch müsste für einen solchen Vorgang ein anderes Material für den Pressstempel 10 verwendet werden, was wiederum den Spannungen beim Pressen nicht zuträglich ist. Auch soll nicht ausgeschlossen sein, dass der Geber 24 des Keramikpressofens 20 direkt auf die Ladung 21 presst, wodurch in der erfindungsgemäßen Vorrichtung nach Anspruch 32 der Pressstempel 10 überflüssig wäre.

Um alle Aspekte der Erfindung in dieser Schrift zu offenbaren, werden im Folgenden noch wichtige Punkte der Erfindung angeführt.

Generell soll nicht ausgeschlossen werden, die erfindungsgemäße Vorrichtung samt Muffel und Pressstempel auch für die Herstellung von Zahnersatzteilen für Tiere, die ein größeres Gebiss als Menschen haben, bereitzustellen.

Für die Einbettmasse kann beispielsweise Siliziumdioxid, Magnesiumoxyd, Amoniumphospat o. Ä. verwendet werden. Die beiden Formgeber bzw. die jeweiligen Formgeber können aus einem Kunststoff (beispielsweise aus Polyoxymethylen) bestehen. Der Silikonring aus Silikon und das rückstandslos ausbrennbare Medium aus Wachs. Für das Ausbrennen des Mediums wird die Lost-Wax-Technik verwendet.

Generell ist die erfindungsgemäße Muffel (die im konkreten erfindungsgemäßen Ausführungsbeispiel ein Gewicht zwischen 300 g und 600 g hat) für alle bekannten herkömmlichen Brennöfen mit hydraulischem Öffnungsmechanismus oder mit Schwenkmechanismus besonders durch das spezielle Design geeignet. Diese Art der Muffel ist auch in der neuen Brennofengeneration mit wesentlich kleinerer Brennkammer für bessere Brandführung geeignet. Das spezielle eiformähnliche Design verhindert bzw. minimiert das Auftreten von Spannungsrissen. Durch die Verwendung gleicher Materialien für Muffel und Pressstempel wird ebenfalls das Auftreten von Spannungsrissen minimiert. Besonders von Vorteil ist die erfindungsgemäße Muffel für das Pressen von zirkulären (hufeneisenförmigen) Brücken. Im Gegensatz zum Pressmaster keine Einwegaufsteckhütchen erforderlich. Durch die spezielle Ausführung über die zwei kegelstumpfförmigen Bereiche hält die Muffel einem wesentlich höheren Anpressdruck als vergleichbare Muffel stand. Ebenfalls ist der Arbeitsaufwand viel geringer, da die Muffel und der Pressstempel in einem Stück herstellbar sind und die gleiche Ausdehnung aufweisen.

Besonders in Anbetracht der vielen Presskanäle, die für die Herstellung eines zirkulär überpressten Metallgerüsts unbedingt notwendig sind, ist das erfindungsgemäße Muffel Pressstempel-System besondert gut geeignet. Besonders im Over-Press-Verfahren wird eine extrem gute Randschlusspassung (Keramikstufen) erreicht.

Aus der Sicht der Zahnmedizin ist die erfindungsgemäße Lösung sowohl für Zahntechniker als auch für Zahnärzte von großem Interesse, da sich auf diese Weise komplexe Patientenfälle mit großspannigen Brückenkonstruktionen in der Überpresstechnik zum ersten Mal versorgen lassen.

## Patentansprüche

1. Muffel zur Herstellung von Zahnersatzteilen, wobei die Muffel (1) ein um die Muffelachse im Wesentlichen rotationssymmetrischer Körper ist, wobei ein auf die gesamte Oberfläche der äußeren Kontur (3) der Muffel (1) bezogener zylindrischer Anteil unter 30 % liegt,
**dadurch gekennzeichnet, dass** die Muffel (1) eine im Wesentlichen konvexe äußere Kontur (3) hat, die von einem unteren zylindrischen Bereich (31) und mindestens zwei sich daran anschließenden kegelstumpfförmigen Bereichen (32a, 32b) gebildet ist.

2. Muffel nach Anspruch 1,
**dadurch gekennzeichnet, dass** die mindestens zwei kegelstumpfförmigen Bereiche (32a, 32b) unterschiedliche Öffnungswinkel (α, β) aufweisen.

3. Muffel nach einem der Ansprüche 1 bis 2,
**dadurch gekennzeichnet, dass** die Muffel (1) einen gekrümmten Oberflächenbereich (33) aufweist, der sich an dem zylindrischen Bereich (31) anschließt.

4. Muffel nach einem der vorhergehenden Ansprüche,
**dadurch gekennzeichnet, dass** der zylindrische Anteil unter 25 %, insbesondere unter 20 %, bevorzugt unter 15 % und besonders bevorzugt unter 10 %, liegt.

5. Muffel nach einem der vorhergehenden Ansprüche,
**dadurch gekennzeichnet, dass** die äußere Kontur (3) der Muffel (1) im oberen (34) und unteren Bereich (35) abgeflacht ist, vorzugsweise zwischen 5 und 55 %, besonders bevorzugt zwischen 30 und 45 %, der äußeren Kontur (3) in rechtem Winkel zur Muffelachse (A) ausgebildet sind.

6. Muffel nach einem der vorhergehenden Ansprüche,
**dadurch gekennzeichnet, dass** der rotationssymmetrische Körper der Muffel (1) - von oben nach unten gesehen -
einen kegelstumpfförmigen Körper (32a') mit einem Öffnungswinkel (α),
einen kegelstumpfförmigen Körper (32b') mit einem Öffnungswinkel (β), wobei α > β gilt,
einen zylindrischen Körper (31') und
einen rotationssymmetrischen Körper mit gekrümmter Oberfläche (33') aufweist.

7. Muffel nach Anspruch 6,
**dadurch gekennzeichnet, dass** die Muffel (1) einen weiteren als Sockel (36) ausgeführten zylindrischen Körper (31') aufweist, der vorzugsweise im unteren Bereich (35) der Muffel (1) angeordnet ist.

8. Vorrichtung zur Herstellung einer Muffel nach einem der Ansprüche 1 bis 7,
**dadurch gekennzeichnet, dass** ein eine Negativform bildender Hohlraum (5a) der Muffel (1) eine flüssige aushärtbare Einbettmasse (4) aufnimmt.

9. Verfahren zur Herstellung von Zahnersatzteilen, wobei in einem Vorwärmofen (19) eine Muffel (1), deren äußere Kontur im Wesentlichen konvex ausgebildet ist und zumindest einen zylindrischen Bereich (31) und mindestens zwei sich daran anschließende kegelstumpfförmige Bereiche (32a, 32b) aufweist, und ein Presstempel (10) erhitzt wird, wobei die Muffel (1) und der Pressstempel (10) aus derselben Einbettmasse (4) bestehen, und wobei eine Ladung (21) für das Zahnersatzteil (2) in einen Aufnahmeraum (23) der Muffel (1) gegeben wird, der Aufnahmeraum (23) samt Ladung (21) mit dem Pressstempel (10) bedeckt wird, die Muffel (1), die Ladung (21) und der Pressstempel (10) in einen Keramikpressofen (20), welcher ein Vakuum erzeugt, gegeben wird, die Ladung (21) durch den Pressstempel (10) in eine durch das Medium (8) in der Muffel (1) gebildete Negativform gepresst wird.

10. Vorrichtung zur Herstellung von Zahnersatzteilen mit einer Muffel, deren äußere Kontur im Wesentlichen konvex ausgebildet ist und zumindest einen zylindrischen Bereich (31) und mindestens zwei kegelstumpfförmige Bereiche (32a, 32b) aufweist, und einem Presstempel (10), wobei in der Muffel eine Negativform der Zahnersatzteile und ein Aufnahmeraum für eine zu verpressende Ladung, insbesondere für eine Zahnersatzteilmasse, ausgebildet sind, und wobei der Aufnahmeraum ein Volumen von über 11 cm³, vorzugsweise von über 12,5 cm, insbesondere ein Volumen zwischen 15 cm³ und 30 cm³, vorzugsweise zwischen 17 cm³ und 23 cm³ aufweist.

## Claims

1. A muffle for the production of dental prostheses, said muffle (1) being a body that Is substantially ratationally symmetrical about the muffle axis, wherein a cylindrical proportion - relative to the total surface area of the outer contour (3) of the muffle (1) - Is less than 30%, **characterized in that** the muffle (1) has a substantially convex outer contour (3) which Is formed by a lower cylindrical region (31) and at least two adjoining frustoconical regions (32a, 32b).

2. The muffle according to claim 1, **characterized in that** the at least two frustoconical regions (32a, 32b) have different cone angles (α, β).

3. The muffle according to one of claims 1 to 2, **characterized In that** the muffle (1) has a curved surface region (33) which adjoins the cylindrical region (31).

4. The muffle according to any of the preceding claims, **characterized in that** the cylindrical proportion is less than 25%, in particular less than 20%, preferably less than 15% and particularly preferably less than 10%.

5. The muffle according to any of the preceding claims, **characterized in that** the outer contour (3) of the muffle (1) is flattened in the upper region (34) and lower region (35), preferably between 5 and 55%, particularly preferably between 30 and 45%, of the outer contour (3) Is formed at a right angle to the muffle axis (A).

6. The muffle according to any of the preceding claims, **characterized in that** the rotationally symmetrical body of the muffle (1) - as viewed from top to bottom - has
a frustoconical body (32a') with a cone angle (a),
a frustoconical body (32b') with a cone angle (b), where α > β,
a cylindrical body (31') and
a rotationally symmetrical body with a curved surface (33').

7. The muffle according to claim 6, **characterized in that** the muffle (1) has a further cylindrical body (31') preferably arranged In the lower region (35) of the muffle (1) and configured as a base (36).

8. A device for producing a muffle according to one of claims 1 to 7, **characterized in that** a cavity (5a) of the muffle (1) forming a negative form receives a liquid curable embedding compound (4).

9. A method of producing dental prostheses, wherein In a pre-heating furnace (19) a muffle (1), whose outer contour Is substantially configured in a convex manner and which has at least one cylindrical region (31) and at least two adjoining frustoconical regions (32a, 32b), and a plunger (10) are heated, wherein the muffle (1) and the plunger (10) consist of the same embedding compound (4), and wherein a charge (21) for the dental prosthesis (2) is put into a receiving space (23) of the muffle (1), the receiving space (23) along with the charge (21) Is covered with the plunger (10), the muffle (1), the charge (21) and the plunger (10) are put into a ceramic pressing furnace (20) which produces a vacuum, the charge (21) Is pressed into a negative form by the plunger (10), said negative form being formed in the muffle (1) by the medium (8).

10. A device for producing dental prostheses using a muffle, whose outer contour is substantially configured in a convex manner and which has at least one cylindrical region (31) and at least two frustoconical regions (32a, 32b), and a plunger (10), wherein a negative form of the dental prostheses and a receiving space for a charge to be pressed, in particular for a dental prosthesis compound, are configured in the muffle, and wherein the receiving space has a volume of more than 11cm³, preferably of more than 12.5cm³, in particular a volume of between 15cm³ and 30 cm³, preferably of between 17cm³ and 23cm³.

## Revendications

1. Moufle pour la fabrication de prothèses dentaires, où le moufle (1) est essentiellement un corps à symétrie de rotation autour de l'axe du moufle, où la partie cylindrique par rapport à toute la surface du contour extérieur (3) du moufle (1) est inférieure à 30 %,
**caractérisé en ce que** le moufle (1) a un contour externe (3) essentiellement convexe, qui est formé par une zone cylindrique inférieure (31) et au moins deux zones (32a, 32b) tronconiques s'y rattachant.

2. Moufle (1) selon la revendication 1, **caractérisé en ce que** les au moins deux zones tronconiques (32a, 32b) présentent des angles d'ouverture (α, β) différents.

3. Moufle (1) selon l'une des revendications 1 ou 2, **caractérisé en ce que** le moufle (1) possède une zone de surface courbé (33), qui se rattache à la zone cylindrique (31).

4. Moufle (1) selon l'une des revendications précédentes, **caractérisé en ce que** la partie cylindrique est inférieure à 25%, en particulier inférieure à 20%, de préférence inférieure à 15% et de façon particulièrement préférée inférieure à 10%.

5. Moufle (1) selon l'une des revendications précédentes, **caractérisé en ce que** le contour extérieure (3) du moufle (1) est aplati dans les zones supérieure (34) et inférieure (35), et composé de préférence entre 5% et 55%, de façon particulièrement préférée entre 30% et 45% du contour extérieur (3) perpendiculairement à l'axe du moufle (A).

6. Moufle (1) selon l'une des revendications précédentes, **caractérisé en ce que** le corps à symétrie de rotation du moufle (1) - vu de haut en bas - présente
un corps tronconique (32a') avec un angle d'ouverture (α),
un corps tronconique (32b') avec un angle d'ouverture (β), où
α> β est vrai,
un corps cylindrique (31') et un corps à symétrie de rotation avec une surface courbée (33').

7. Moufle (1) selon la revendication 6, **caractérisé en ce que** le moufle (1) possède un autre corps cylindrique (31'), réalisé comme socle (36), qui est disposé de préférence dans la zone inferieure (35) du moufle (1).

8. Dispositif pour la fabrication d'un moufle selon l'une des revendications 1 à 7, **caractérisé en ce qu'**une cavité (5a) du moufle (1) formant un moule négatif accueille une masse d'enrobage liquide durcissable (4).

9. Procédé pour la fabrication de prothèses dentaires, où un moufle (1), dont le contour externe est formé essentiellement convexe et possède au moins une surface cylindrique (31) et au moins deux zones tronconiques (32 a, 32 b) s'y rattachant, et une matrice de pressage (10) est chauffé dans un four de préchauffage (19), où le moufle (1) et la matrice de pressage (10) sont composés de la même masse d'enrobage (4), et où une charge (21) pour la prothèse dentaire (2) est introduite dans un espace de réception (23) du moufle (1), l'espace de réception (23) ainsi que de la charge (21) est couvert de la matrice de pressage (10), le moufle (1), la charge (21) et la matrice de pressage (10) est introduit dans un four à céramique pressée(20), qui crée un vide, la charge (21) (1) est pressé à travers la matrice de pressage (10) dans un moule négatif formé par le milieu (8) dans le moufle.

10. Dispositif pour la fabrication de prothèses dentaires avec un moufle, dont le contour extérieur est réalisé essentiellement convexe et possède au moins une zone cylindrique (31) et au moins deux zones tronconiques (32a, 32b) et d'une matrice de pressage (10), où un moule négatif des prothèses dentaires et un espace de réception pour une charge à presser, en particulier pour une masse pour prothèse dentaire, sont réalisés dans le moufle, et où l'espace de réception présente un volume de 11 cm³, de préférence supérieur à 12,5 cm³, en particulier, un volume compris entre 15 cm³ et 30 cm³, de préférence entre 17 cm³ et 23 cm³.
